(19) Europäisches Patentamt
European Patent Office
Office européen des brevets

(11) Numéro de publication: **0 148 096**
**B1**

(12) # FASCICULE DE BREVET EUROPEEN

(45) Date de publication du fascicule du brevet: **05.08.87**

(51) Int. Cl.⁴: **C 07 C 91/12**, A 61 K 31/13 // C07D303/36

(21) Numéro de dépôt: **84450025.6**

(22) Date de dépôt: **13.11.84**

(54) **Nouveaux arylamino-1 amino-3 propanols-2 diversement subtitués, leur méthode de préparation ainsi que leur application en thérapeutique.**

(30) Priorité: **22.12.83 FR 8320747**

(43) Date de publication de la demande: **10.07.85 Bulletin 85/28**

(45) Mention de la délivrance du brevet: **05.08.87 Bulletin 87/32**

(84) Etats contractants désignés: **BE CH DE GB IT LI LU NL**

(56) Documents cités:
**CHEMICAL ABSTRACTS, vol. 15. no. 12, 20 juin 1921, page 1885, Columbus Ohio, US; E. FOURNEAU et al.: "Amino alcohols derived from alpha-anilino-upsilon-dialkylaminoisopropyl alcohols"**
**CHEMICAL ABSTRACTS, vol. 70, no. 9, 3 mars 1969, page 296, abrégé 37343k, Columbus, Ohio, US; S. KUTKEVICIUS et al.: "N-(upsilon-Chloro-beta-hydroxylpropyl)arylamines and their reaction products. VI. N-mono- and N,N-bis(beta,upsilon-epoxypropyl)amines"**
**CHEMICAL ABSTRACTS, vol. 69, 1968, page 7170, abrégé 76809r, Columbus, Ohio, US; G. FERRARI et al.: "Beta-Adrenergic blocking drugs. III. 1-Aryloxy- and 1-arylamino-3-amino-2-propanols",**

**Le dossier contient des informations techniques présentées postérieurement au dépôt de la demande et ne figurant pas dans le présent fascicule.**

(73) Titulaire: **SOCIETE CORTIAL S.A., 7 rue de l'Armorique, F-75015 Paris (FR)**

(72) Inventeur: **Boyer, Chantal, 18 route de Soulac Beaulieu, F-33290 Le Pian Médoc (FR)**
Inventeur: **Colleter, Jean-Claude, Rue du Général de Gaulle Ludon Médoc, F-33290 Blanquefort (FR)**
Inventeur: **Creuzet, Marie-Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Laguerre, Michel, Résidence La Fleurière Bât A Appt 15, F-33170 Gradignan (FR)**
Inventeur: **Pontagnier, Henri, 21 rue Edouard Vaillant, F-33600 Pessac (FR)**
Inventeur: **Prat, Gisèle, Hameau de Noailles Villa 18, F-33400 Talence (FR)**

(74) Mandataire: **Tajan, Marie-Thérèse, LABORATOIRES SARGET Avenue du Président JF Kennedy, F-33701 Mérignac (FR)**

EP 0 148 096 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux arylamino-1 amino-3 propanols-2 diversement substitués, leur méthode de préparation ainsi que leur application en thérapeutique notamment dans le traitement des troubles du rythme.

Les produits faisant l'objet de la présente invention ont pour formule générale

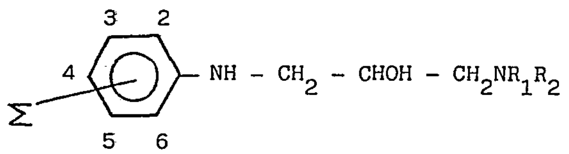

avec Σ représentant un groupement isopropyl en position 2 ou un groupement méthyl en position 4 ou un groupement isopropyl en position 2 et un groupement méthyl en position 6, et avec $NR_1R_2$ représentant un groupement NHtertiobutyl, un groupement NHisopropyl ou un groupement $N(CH_3)_2$ sous réserve que $NR_1R_2$ soit différent de $N(CH_3)_2$ si Σ représente un groupement méthyl en position 4. Ces produits peuvent être sous forme de base libre ou de sels pharmaceutiquement compatibles tels que leurs chlorhydrates.

On connaît déjà des arylamino-1 amino-3 propanols-2 diversement substitués. Ainsi FERRARI G., FERRINI R. et CASAGRANDE C. ont décrit (Boll. Chim. Farm., 1968, 107, 234-247) des arylamino-1 amino-3 propanols-2 et ont exposé leurs propriétés bêtabloquantes et anesthésiques locales. Les propriétés bêtabloquantes de ces dérivés sont soit très faibles soit très inférieures à celles de leurs homologues aryloxy-1 et n'ont donc pas été étudiées plus précisément par ces auteurs. SINHA J.N., RASTOGI V.K. et PARMAR S.S. décrivent l'activité bêta$_2$ bloquante spécifique du (diméthyl-2,4 anilino)-1 isopropylamino-3 propanol-2 (Eur. J. Pharmacol., 1972, 19, 285-287).

Nous avons maintenant découvert que les nouveaux arylamino-1 amino-3 propanols-2 qui font l'objet de la présente invention présentent des propriétés antiarythmiques permettant leur emploi en thérapeutique dans le traitement des troubles du rythme. Ils présentent également des activités anesthésiques locales, antiagrégantes plaquettaires, anticalciques, hypolipidémiantes, diurétiques, antihistaminique H1 et antihistaminiques H2 permettant leur emploi en thérapeutique dans diverses indications.

Les produits de la présente invention sont préparés de façon générale à partir de l'arylamine

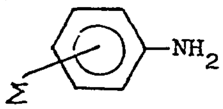

(avec Σ tel que défini plus haut), qui par réaction avec l'épichlorhydrine

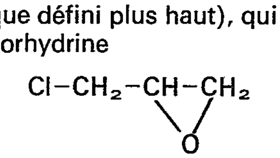

dans un solvant tel que l'éthanol à la température d'ébullition du solvant, conduit au chlorhydrate de

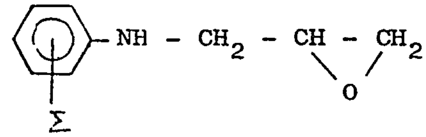

Ce chlorhydrate en présence de l'amine $NHR_1R_2$ dans un solvant tel que l'éthanol à une température égale ou supérieure à la température d'ébullition du solvant conduit au produit de formule (I)

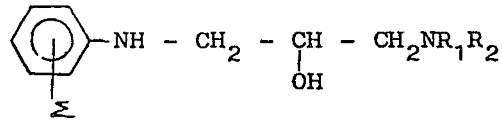

avec Σ, $R_1$ et $R_2$ tels que définis plus haut.

Les exemples ci-dessous vont préciser la présente invention sans toutefois en limiter la portée.

*Exemple 1:*

Dichlorhydrate de l'(isopropyl-2 anilino)-1 tertiobutylamino-3 propanol-2 ou COR37 05C. Dichlorhydrate du produit de formule (I) avec Σ = iPr-2, $NR_1R_2$ = NHtBu.

On porte au reflux dans de l'éthanol absolu pendant 24 heures une mole d'isopropyl-2 aniline et 1,1 mole d'épichlorhydrine. On obtient ainsi le chlorhydrate de

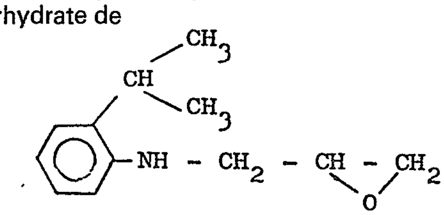

Celui-ci est mis en solution dans l'éthanol absolu et porté au reflux pendant 24 heures en présence d'un grand excès de tertiobutylamine. Après évaporation des solvants, le produit attendu est purifié par colonne sur alumine ou silice et recristallisé. La base est ainsi obtenue avec un rendement de 70% puis est transformée en dichlorhydrate ou COR37 05C.

*Caractéristiques physico-chimiques:*

Point de fusion = 140°C.

Point de fusion de la base correspondante = 82°C.

Spectre de RMN de la base/TMS: 1 s (9 H) à 1,06 ppm, tBu; 1 d (6 H) à 1,25 ppm, J = 7 Hz, $CMe_2$; 1 m (2 H) à 2,70 ppm, $CH_2$ N(tBu); 1 m (3 H) à 3,2 ppm; Ar N $CH_2$ + Ar CH $Me_2$: 1 m (1 H) à 3,8 ppm, CHO; 1 m (2 H) à 6,7 ppm, 2 protons aromatiques; 1 m (2 H) à 7,1 ppm, 2 protons aromatiques.

*Exemple 2:*

Dichlorhydrate de l'(isopropyl-2 méthyl-6 anilino)-1 tertiobutylamino-3 propanol-2 ou COR37 06C. Dichlorhydrate du produit de formule (I) avec Σ = iPr-2 Me-6, $NR_1R_2$ = NHtBu.

On porte au reflux dans de l'éthanol absolu pendant 24 heures 1 mole d'isopropyl-2 méthyl-6 aniline et 1,1 mole d'épichlorhydrine. On obtient ainsi le chlorhydrate de

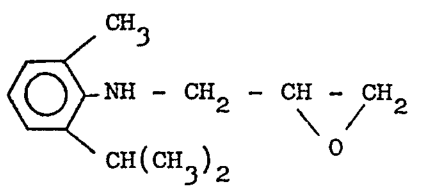

Celui-ci est mis en solution dans l'éthanol absolu et porté au reflux pendant 24 heures en présence d'un grand excès de tertiobutylamine. Après évaporation des solvants le produit attendu est purifié par colonne sur alumine ou silice et recristallisé. La base est ainsi obtenue avec un rendement de 70% puis transformée en dichlorhydrate ou COR37 06C.

*Propriétés physico-chimiques:*

Point de fusion = 158°C.

Spectre de RMN de la base/TMS: 1 s (9 H) à 1,1 ppm, tBu; 1 d (6 H) à 1,25 ppm, J = 7 Hz, $CMe_2$; 1 s (3 H) à 2,3 ppm Ar $CH_3$; 1 m (2 H) à 2,7 ppm, $\underline{CH_2}$ NH tBu; 1 m (2 H) à 3,0 ppm, Ar $NCH_2$; 1 sp (1 H) à 3,2 ppm, Ar $\underline{CH}Me_2$; 1 m (1 H) à 3,8 ppm, CHO. 1 m (3 H) à 6,9 ppm, 3 protons aromatiques.

*Exemple 3:*

Dichlorhydrate du (méthyl-4 anilino)-1 tertiobutylamino-3 propanol-2 ou COR37 09C. Dichlorhydrate du produit de formule (I) avec Σ = Me-4, $NR_1R_2$ = NHtBu.

On porte au reflux dans de l'éthanol absolu pendant 24 heures 1 mole de méthyl-4 aniline et 1,1 mole d'épichlorhydrine. On obtient ainsi le chlorhydrate de

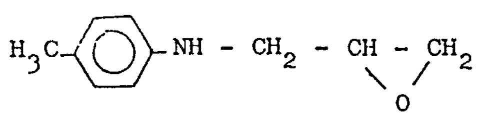

Celui-ci est mis en solution dans l'éthanol absolu et porté au reflux pendant 24 heures en présence d'un grand excès de tertiobutylamine. Après évaporation des solvants le produit attendu est purifié par colonne sur alumine ou silice et recristallisé. La base est obtenue avec un rendement de 60% puis transformée en dichlorhydrate ou COR37 09C.

*Propriétés physico-chimiques:*

Point de fusion de la base correspondante = 82°C.

Spectre de RMN de la base/TMS: 1 s (9 H) à 1,1 ppm, tBu; 1 s (3 H) à 2,2 ppm, Ar $CH_3$; 1 m (2 H) à 2,6 ppm, $\underline{CH_2}$ NHtBu; 1 m (2 H) à 3,1 ppm, Ar $NCH_2$; 1 m (1 H) à 3,7 ppm, CHO. Spectre AB (4 H) à 6,5 et 6,95 ppm, J = 8 Hz, 4 protons aromatiques.

*Exemple 4:*

Dichlorhydrate de l'(isopropyl-2 anilino)-1 diméthylamino-3 propanol-2 ou COR37 49C. Dichlorhydrate du produit de formule (I) avec Σ = iPr-2, $NR_1R_2 = N(CH_3)_2$.

Le chlorhydrate de

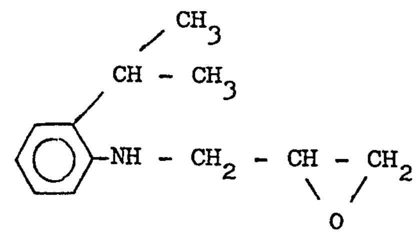

préparé selon l'exemple 1 est mis en solution dans l'éthanol absolu et maintenu à l'autoclave à une température de 100°C pendant 24 heures en présence d'un grand excès de diméthylamine. Après évaporation des solvants le produit attendu est purifié par colonne sur alumine ou silice et recristallisé. La base est obtenue avec un rendement de 47% puis transformée en dichlorhydrate ou COR37 49C.

*Propriétés physico-chimiques:*

Spectre de RMN de la base/TMS: 1 d (6 H) à 1,15 ppm, J = 6 Hz, $C(CH_3)_2$; 1 s (6 H) à 1,95 ppm, $N(CH_3)_2$; 1 m (5 H) de 2,5 à 3,3 ppm, $CH(CH_3)_2 + 2\,CH_2N$; 1 m (1 H) à 3,8 ppm, CHO; 1 m (2 H) à 6,5 ppm, 2 protons aromatiques; 1 m (2 H) à 6,95 ppm, 2 protons aromatiques.

*Exemple 5:*

Dichlorhydrate de l'(isopropyl-2 méthyl-6 anilino)-1 diméthylamino-3 propanol-2 ou COR37 50C. Dichlorhydrate du produit de formule (I) avec iPr-2 Me-6, $NR_1R_2 = N(CH_3)_2$.

Le chlorhydrate de

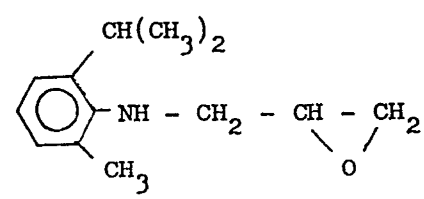

préparé selon l'exemple 2 est mis en solution dans l'éthanol absolu et maintenu à l'autoclave à une température de 110°C pendant 24 heures en présence d'un grand excès de diméthylamine. Après évaporation des solvants le produit attendu est purifié par colonne sur alumine ou silice et recristallisé. La base est obtenue avec un rendement de 42% puis transformée en dichlorhydrate ou COR37 50C.

*Propriétés physico-chimiques:*

Spectre de RMN de la base/TMS: 1 d (6 H) à 1,25 ppm, J = 6 Hz, $C(CH_3)_2$; 1 s (9 H) à 2,25 ppm, Ar $\underline{CH_3} + N(CH_3)_2$; 1 m (5 H) de 2,5 à 3,5 ppm, $\underline{CH}(CH_3)_2 + 2NCH_2$; 1 m (1 H) à 3,85 ppm, $\overline{CHO}$; 1 m (3 H) à 7,0 ppm, 3 protons aromatiques.

Les propriétés toxicopharmacologiques des produits faisant l'objet de la présente invention sont exposées ci-après.

La toxicité a été déterminée chez la souris. En solution dans le Tween et après administration par voie orale le COR37 05C entraîne 0% de mortalité à 500 mg/kg, 40% à 750 et 40% à 1000; le COR37 06C entraîne 0% de mortalité à 100 mg/kg, 10% à

200, 10% à 250, 90% à 375, 100% à 500 (DL50 = 294 mg/kg) (261-332); les COR37 09C, COR37 49C et COR37 50C entraînent 0% de mortalité à 300 mg/kg. En solution dans le Tween et après administration par voie intrapéritonéale le COR37 05C entraîne 0% de mortalité à 200 mg/kg, le COR37 06C entraîne 0% de mortalité à 100 mg/kg et 100% à 200, le COR37 09C entraîne 0% de moralité à 50 mg/kg et 100% à 100 mg/kg, les COR37 49C et 50C n'entraînent aucune mortalité à 200 mg/kg. Par voie intraveineuse le COR37 05C présente une DL50 de 56,6 mg/kg (53,8-59,5).

L'activité antiarythmique a été déterminée sur plusieurs modèles expérimentaux. Dans le modèle de la souris soumise à une anesthésie chloroformique profonde le COR37 05C et le COR37 06C présentent après administration intrapéritonéale une activité antiarythmique dès la dose de 25 mg/kg; le COR37 50C est actif sur ce même modèle à 100 mg/kg. Le propanolol est actif dans les mêmes conditions à la dose de 40 mg/kg et la quinidine à la dose de 100 mg/kg.

In vitro dans le modèle de l'oreillette isolée de cobaye stimulée électriquement le COR37 05C et le COR37 06C sont actifs avec des Ci30 de respectivement 6,22 (4,37-8,86) $10^{-6}$ M/l et $7,26 \cdot 10^{-6}$ ($4,68 \cdot 10^{-6}$-$1,12 \cdot 10^{-5}$) M/l contre $10^{-5}$ M/l pour la procaïne, $4,10^{-6}$ M/l pour la quinidine et $4,5 \cdot 10^{-6}$ pour le propanolol.

Ces produits ont été testés dans le modèle des arythmies induites par l'aconitine chez le rat. Les résultats sont exprimés par le pourcentage d'augmentation de la quantité d'aconitine provoquant l'apparition d'extrasystoles (E), de fibrilloflutters (F), de tachycardies (T) et de morts (M). Administrés par voie intraveineuse 1 min avant la perfusion d'aconitine,le COR37 05C entraîne à 10 mg/kg 78% (E), 87% (T) et 83% (M) d'activité et le COR37 06C à 5 mg/kg entraîne 45% (E), 39% (T) et 79% (M) d'activité. Administrés par voie orale 45 min avant la perfusion d'aconitine à 100 mg/kg, le COR37 05C entraîne 83% (E), 99% (T), 43% (F) et 107% (M) d'activité et le COR37 06C 25% (E), 32% (T), 29% (F) et 105% (M) d'activité. De plus le pourcentage d'animaux ne présentant pas de fibrilloflutters après administration de 100 mg/kg de COR37 05C et COR37 06C par voie orale est respectivement de 70 et 60%.

L'activité anesthésique locale a été déterminée dans le test de la cornée de cobaye soumise à un stimulus tactile. Le COR37 06C employé à la concentration de 0,5% est actif pendant 30 min.

L'activité antiagrégante plaquettaire a été déterminée in vitro. Quand l'agrégation est induite par 0,05 ml d'une préparation standardisée de collagène bovin dans un plasma riche en plaquettes (PRP) de lapin, le COR37 06C entraîne 100% d'inhibition à 1 microg/ml alors que l'aspirine dans les mêmes conditions est active à 10 microg/ml et l'indométacine à 0,5 microg/ml. Dans le modèle de l'agrégation plaquettaire induite par $10^{-6}$ M d'ADP dans un PRP de lapin, 100 microg/ml, le COR37 09C entraîne 79% d'activité, le COR37 50C entraîne 80% d'activité et l'adénosine 64%.

Les produits de la présente invention ne présentent pas d'activité bêtabloquante. Celle-ci a été déterminée in vitro par la recherche sur oreillette de cobaye stimulée électriquement d'un blocage de l'action inotrope de l'isoprotérénol et in vivo par la recherche de l'inhibition de la tachycardie induite par l'isoprotérénol chez la souris ainsi que par la recherche de l'activité vis-à-vis des effets de l'isoprénaline sur la pression artérielle et la fréquence cardiaque du rat normal anesthésié. Le COR37 05C n'est actif que vis-à-vis des récepteurs bêta2 adrénergiques; les COR37 06C, COR37 09C, COR37 49C et COR37 50C ne présentent pas de propriétés bêtabloquantes.

L'activité anticalcique a été déterminée in vitro sur oreillette gauche de cobaye stimulée électriquement à 150 battements/min dans une solution de Tyrode pauvre en calcium (0,6 mM). Les COR37 49C et 50C utilisés à la concentration de 100 microg/ml inhibent l'augmentation de la force contractile induite par l'addition de 0,6 mM de calcium.

L'activité hypolipidémiante a été mise en évidence chez la souris rendue hypercholestérolémique par un régime riche en cholestérol et acide cholique pendant 7 jours. Ainsi l'administration par voie orale du COR37 49C le 6$^{e}$ et le 7$^{e}$ jour entraîne une diminution du cholestérol sérique de 22% à la dose de 400 mg/kg.

L'activité diurétique a été déterminée chez le rat. Ainsi le COR37 49C administré au rat en surcharge hydrique à la dose orale de 20 mg/kg multiplie par 2,2 l'excrétion urinaire de sodium.

L'activité antihistaminique antiH2 a été mise en évidence in vitro. A la concentration de 25 microg/ml le COR37 49C inhibe à 50% l'effet chronotrope induit par 5 microg/ml d'histamine sur l'oreillette droite de cobaye non stimulée.

L'activité antihistaminique antiH1 a été mise en évidence in vitro sur iléon de cobaye. Le COR37 49C employé à la concentration de 25 microg/ml inhibe à 80% les contractions induites par l'histamine.

Compte tenu de leurs activités pharmacologiques jointes à une toxicité peu élevée, les produits faisant l'objet de la présente invention peuvent être employés en thérapeutique humaine et vétérinaire. Associés aux excipients habituels, ils pourront être utilisés par exemple pour traiter les troubles du rythme cardiaque. Ils pourront également être employés dans l'anesthésie locale d'infiltration et de conduction et dans l'anesthésie de contact, dans le traitement des états d'hyperagrégabilité plaquettaire, dans le traitement de l'insuffisance coronarienne, dans le traitement de l'hyperlipémie, l'hyperlipoprotéinémie et des états œdémateux, dans le traitement de la maladie ulcéreuse et dans le traitement des affections allergiques ou prurigineuses et des maux de transport.

Ils seront administrés par exemple par voie orale sous forme de dragées, comprimés, sirop, ampoules, par voie rectale sous forme de suppositoires, par voie intramusculaire ou intraveineuse, ou bien par voie topique sous forme de pommade ou gel. Les doses administrées varieront selon l'indication

et le sujet de 1 à 100 mg/j en une à six prises pour la voie orale, de 1 à 100 mg/j en une ou deux prises par la voie rectale, de 0,5 à 50 mg par injection pour les voies parentérales.

## Revendications

1. Nouveaux produits de formule générale

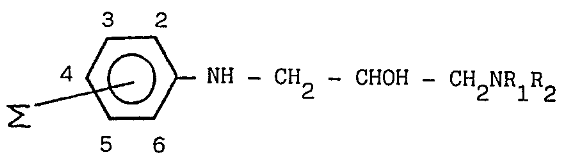

avec Σ représentant un groupement isopropyl en position 2 ou un groupement méthyl en position 4 ou un groupement isopropyl en position 2 et un groupement méthyl en position 6, et avec $NR_1R_2$ représentant un groupement NHtertiobutyl, un groupement NHisopropyl ou un groupement $N(CH_3)_2$ sous réserve que $NR_1R_2$ soit différent de $N(CH_3)_2$ si Σ représente un groupement méthyl en position 4, sous forme de bases libres ou de sels pharmaceutiquement compatibles.

2. Nouveaux produits selon la revendication 1, caractérisés en ce que $NR_1R_2$ représente NHtertiobutyl.

3. Nouveaux produits selon la revendication 1, caractérisés en ce que $NR_1R_2$ représente $N(CH_3)_2$ sous réserve que $NR_1R_2$ soit différent de $N(CH_3)_2$ si Σ représente un groupement méthyl en position 4.

4. Nouveaux produits selon les revendications 1 et 2, caractérisés en ce qu'ils sont constitués par l'(isopropyl-2 anilino)-1 tertiobutylamino-3 propanol-2 sous forme de base libre ou de sels pharmaceutiquement compatibles.

5. Nouveaux produits selon les revendications 1 et 2, caractérisés en ce qu'ils sont constitués par l'(isopropyl-2 méthyl-6 anilino)-1 tertiobutylamino-3 propanol-2 sous forme de base libre ou de sels pharmaceutiquement compatibles.

6. Méthode de préparation des produits selon les revendications 1 à 5, caractérisée en ce que l'on fait réagir, dans un solvant tel que l'éthanol, à la température d'ébullition du solvant, une arylamine de formule générale avec Σ représentant un groupement isopropyl en position 2 ou un groupement méthyl en position 4 ou un groupement isopropyl en position 2 et un groupement méthyl en position 6, avec l'épichlorhydrine

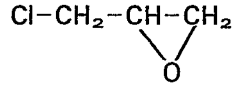

et en ce que l'on fait réagir le chlorhydrate ainsi obtenu avec l'amine $NHR_1R_2$ (où $NR_1R_2$ représente un groupement NHtertiobutyl, un groupement NHisopropyl ou un groupement $N(CH_3)_2$), sous réserve que $NR_1R_2$ soit différent de $N(CH_3)_2$ si Σ représente un groupement méthyl en position 4, dans un solvant tel que l'éthanol à des températures supérieures ou égales à la température d'ébullition du solvant.

7. Nouveau médicament, caractérisé en ce que le principe actif est constitué par au moins un produit selon les revendications 1 à 5.

8. Nouveau médicament selon la revendication 7, utile pour le traitement des troubles du rythme cardiaque.

9. Nouveau médicament selon la revendication 7, utile pour le traitement des états d'hyperagrégabilité plaquettaire.

10. Nouveau médicament selon la revendication 7, utile dans l'anesthésie.

11. Nouveau médicament selon la revendication 7, utile dans le traitement de l'insuffisance coronarienne, de l'hyperlipémie, de l'hyperlipoprotéinémie, des états œdémateux, de la maladie ulcéreuse et des affections allergiques.

## Patentansprüche

1. Neue Verbindungen der allgemeinen Formel

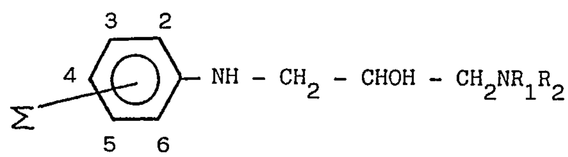

in der Σ eine Isopropylgruppe in Position 2 oder eine Methylgruppe in Position 4 oder eine Isopropylgruppe in Position 2 und eine Methylgruppe in Position 6 bedeutet und $NR_1R_2$ eine NH-tert.-Butyl-Gruppe, eine NH-Isopropyl-Gruppe oder eine $N(CH_3)_2$-Gruppe bedeuten, mit der Bedingung, dass $NR_1R_2$ nicht gleich $N(CH_3)_2$ ist, wenn Σ eine Methylgruppe in Position 4 ist, in Form ihrer freien Basen und pharmazeutisch verträglichen Salze.

2. Verbindungen nach Anspruch 1, dadurch gekennzeichnet, dass $NR_1R_2$ NH-tert.-Butyl bedeutet.

3. Verbindungen nach Anspruch 1, dadurch gekennzeichnet, dass $NR_1R_2$ $N(CH_3)_2$ bedeutet, unter der Bedingung, dass $NR_1R_2$ nicht gleich $N(CH_3)_2$ ist, wenn Σ eine Methylgruppe in Position 4 bedeutet.

4. Verbindungen nach Anspruch 1 oder 2, gekennzeichnet durch (Isopropyl-2-anilino)-1-tert.-butylamino-3-propanol-2 in Form seiner freien Base oder pharmazeutisch verträglichen Salze.

5. Verbindungen nach Anspruch 1 oder 2, gekennzeichnet durch (Isopropyl-2-methyl-6-anilino)-1-tert.-butylamino-3-propanol-2 in Form seiner freien Base oder pharmazeutisch verträglichen Salze.

6. Verfahren zur Herstellung der Verbindungen nach einem der Ansprüche 1 bis 5, dadurch gekennzeichnet, dass man in einem Lösemittel wie Ethanol bei der Siedetemperatur des Lösemittels ein Arylamin der allgemeinen Formel

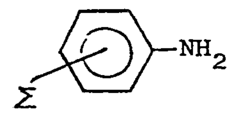

in der Σ eine Isopropylgruppe in Position 2 oder eine Methylgruppe in Position 4 oder eine Isopropylgruppe in Position 2 und eine Methylgruppe in Position 6 bedeutet, mit Epichlorhydrin der Formel

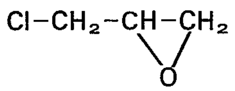

umsetzt, und dass man das so erhaltene Chlorhydrat mit einem Amin $NHR_1R_2$ (wobei $NR_1R_2$ eine NH-tert.-Butyl-Gruppe, eine NH-Isopropyl-Gruppe oder eine $N(CH_3)_2$-Gruppe bedeutet), unter der Bedingung, dass $NR_1R_2$ nicht gleich $N(CH_3)_2$ ist, wenn Σ eine Methylgruppe in Position 4 ist, in einem Lösemittel wie Ethanol bei Temperaturen oberhalb oder gleich der Siedetemperatur des Lösemittels umsetzt.

7. Neues Arzneimittel, dadurch gekennzeichnet, dass der Wirkstoff mindestens eine Verbindung gemäss einem der Ansprüche 1 bis 5 ist.

8. Arzneimittel nach Anspruch 7, für die Behandlung von Herzrhythmusstörungen.

9. Arzneimittel nach Anspruch 7, für die Behandlung von Zuständen einer Hyperagregation der Blutplättchen.

10. Arzneimittel nach Anspruch 7, zur Verwendung in der Anästhesie.

11. Arzneimittel nach Anspruch 7 zur Verwendung bei der Behandlung der Coronarinsuffizienz, der Hyperlipämie, der Hyperlipoproteinämie, von ödemateusen Zuständen, von Geschwürerkrankungen und von allergischen Erkrankungen.

## Claims

1. New products with the general formula

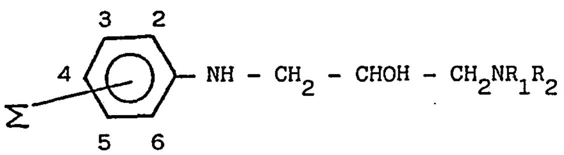

with Σ representing an isopropyl group in position 2 or a methyl group in position 4, or an isopropyl group in position 2 and a methyl group in position 6, and with $NR_1R_2$ representing a NH tertiobutyl group, a NH isopropyl group, or a $N(CH_3)_2$ on the condition that $NR_1R_2$ be different from $N(CH_3)_2$ when Σ represents a methyl group in position 4, in the form of free bases or pharmaceutically-compatible salts.

2. New products as defined in Claim 1, in which $NR_1R_2$ represents NH tertiobutyl.

3. New products as defined in Claim 1, in which $NR_1R_2$ represents $N(CH_3)_2$ on the condition that $NR_1R_2$ be different from $N(CH_3)_2$ when Σ represents a methyl group in position 4.

4. New products as defined in Claims 1 and 2, which are comprised of (isopropyl-2 anilino)-1 tertiobutyl-amino-3 propanol-2, in the form of free base or pharmaceutically-compatible salts.

5. New products as defined in Claims 1 and 2, which are comprised of (isopropyl-2 methyl-6 anilino)-1 tertiobutylamino-3 propanol-2 in the form of free base or pharmaceutically-compatible salts.

6. Method for preparing products as defined in Claims 1 to 5, which comprises reacting in a solvent such as ethanol, at the solvent boiling temperature, an arylamine with said general formula, with Σ representing an isopropyl group in position 2 or a methyl group in position 4, or an isopropyl group in position 2 and a methyl group in position 6, with epichlorhydrin

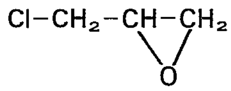

and further reacting the resulting chlorhydrate with the amine $NHR_1R_2$ (where $NR_1R_2$ represents a NH tertiobutyl group, a NH isopropyl group or a $N(CH_3)_2$ group), on the condition that $NR_1R_2$ be different from $N(CH_3)_2$ when Σ represents a methyl group in position 4, in a solvent such as ethanol at temperatures higher than or equal to the solvent boiling temperature.

7. New medicament, in which the active constituent is comprised of at least one product as defined in claims 1 to 5.

8. New medicament as defined in Claim 7, useful for treating heart-rhythm disorders.

9. New medicament as defined in Claim 7, useful for treating plaquette hyper-aggregability conditions.

10. New medicament as defined in Claim 7, useful for anaesthesia.

11. New medicament as defined in Claim 7, useful for treating coronary deficiency, hyperlipaemia, hyperlipoproteinaemia, oedematose conditions, ulcerous illness, and allergy ailments.